# FASCICULE DE BREVET EUROPEEN

(11) **EP 1 786 386 B1**
(45) Date de publication et mention de la délivrance du brevet: **18.08.2010**
(21) Numéro de dépôt: 05778687.3
(22) Date de dépôt: 22.06.2005
(51) Int. Cl.: A61K 8/64, A61Q 19/08

(54) **Utilisation cosmétique, non-thérapeutique, d'au moins un tétrapeptide naturel Ac-N-Ser-Asp-Lys-Pro ou un de ses sels en tant qu'agent antiviellissement et restructurant de la peau**
Nicht-therapeutische, kosmetische Anwendung von mindestens einer Art von natürlichem Ac-N-Ser-Asp-Lys-Pro Tetrapeptid oder seinen Salzen in Form eines Mittels gegen Hautalterung und zur Hautrestrukturierung
Non therapeutic, cosmetic use of at least one type of natural Ac-N-Ser-Asp-Lys-Pro tetrapeptide or its salts in the form of a skin antiaging and restructuring agent

(30) Priorité: 23.06.2004 FR 0406822
(43) Date de publication de la demande: 23.05.2007
(73) Titulaire: CENTRE NATIONAL DE LA RECHERCHE SCIENTIFIQUE (CNRS) Etablissement Public, 75016 Paris (FR)
(72) Inventeur: BAKALA, Joanna, F-75012 Paris (FR); LALLEMAND, Jean-Yves, F-91120 Palaiseau (FR); LIU Jian-Miao, 942 00 Ivry (FR); BIGNON, Jérôme, F-91350 Le Val Saint Germain (FR)
(74) Mandataire: Ahner, Francis
(86) Numéro de dépôt international: PCT/FR2005/001568
(87) Numéro de publication internationale: WO 2006/008392

(56) Documents cités:
- WO-A-02/24218
- US-A- 5 348 943
- US-A- 5 492 894
- PATENT ABSTRACTS OF JAPAN vol. 014, no. 453, 28 septembre 1990 (1990-09-28) & JP 02 178207 A (KANEBO LTD), 11 juillet 1990 (1990-07-11)

## Description

La présente invention concerne l'utilisation cosmétique non thérapeutique dans une composition, d'au moins un composé tétrapeptidique ou d'un de ses sels, en tant qu'agent anti-vieillissement et restructurant de la peau.

La peau est un ensemble de cellules regroupées sous forme d'un tissu résistant et souple, recouvrant la totalité du corps. La peau a pour fonction principale d'établir une barrière de protection contre les atteintes de l'environnement tout en permettant certains échanges entre le milieu intérieur et le milieu extérieur. Elle est le siège de nombreux processus métaboliques qui sont modulés par les conditions physiologiques de l'organisme et les conditions de l'environnement. La peau est formée de deux couches jointes : l'épiderme et le derme auxquelles on peut associer les tissus sous-cutanés.

L'épiderme dont le principal rôle est la protection du corps constitue la couche la plus superficielle de la peau et assure l'imperméabilité de la peau et sa résistance. Il se renouvelle toutes les quatre semaines environ. Si différents types cellulaires coexistent dans l'épiderme, les kératinocytes sont largement majoritaires (90 %). Leur activité caractéristique est la synthèse des kératines qui représentent 95 % des protéines totales de l'épiderme. Les kératines, protéines fibreuses et insolubles dans l'eau, entrent dans la constitution de la couche cornée de l'épiderme qui protège la peau contre les agressions extérieures (chaleur, froid, déshydratation).

L'épiderme est relié au derme par une zone appelée jonction dermo-épidermique ou membrane basale épidermique. Cette structure assure l'adhérence du derme à l'épiderme et joue le rôle de support mécanique responsable en partie de la tonicité de la peau. Elle est élaborée à la fois par les kératinocytes basaux et par les fibroblastes dermiques et elle contient un taux important de collagène de type IV qui fait partie des plaques d'ancrage reliant la membrane basale à des plaques d'ancrage présentes dans le derme papillaire.

Le derme, la couche interne de la peau, est un tissu conjonctif fibro-élastique composé de cellules (les fibroblastes) dispersées dans un milieu complexe appelé matrice extracellulaire. Cette matrice est constituée de fibres de collagène et d'élastine, de glycoprotéines (fibronectine et laminine) et de protéoglycanes (protéine centrale + glycosaminoglycanes ou GAGs). La nature et la quantité de ces composants régissent les propriétés mécaniques de la peau et sont à l'origine des modifications physiopathologiques les plus visibles du relief cutané observées au cours du vieillissement. Les fibres donnent à la peau solidité, résistance, élasticité et tonicité. Les glycoprotéines et protéoglycanes apportent le volume et participent à l'hydratation.

Les collagènes, les protéines composant 75 % du derme, représentent le « ciment » du derme. Leur arrangement spatial particulier confère une rigidité à la molécule de collagène, contribuant ainsi à la fonction mécanique de celle-ci. Le collagène fibrillaire de type III dont la localisation dermique est reconnue, constitue le composant essentiel du réseau fibreux et possède un rôle mécanique en assurant en grande partie le soutien et l'élasticité de la peau.

Les protéoglycanes, macromolécules complexes de grande taille dispersées entre les fibres du tissu conjonctif dermique, assurent en grande partie la tonicité de la peau. En fait, la structure des glycosaminoglycanes (GAGs). polymères formés de longues chaînes de polysaccharides qui font partie de ces macromolécules, confèrent aux protéoglycanes des charges négatives permettant de "piéger" les ions, l'eau et divers métabolites, contribuant ainsi principalement à l'hydratation de la peau et à la résistance aux forces de pression et d'étirement.

En vieillissant, la peau se ride, se relâche, est moins hydratée et se répare plus difficilement. Ces signes cliniques qui affectent l'apparence ou causent des pathologies cutanées résultent de la superposition d'un processus «extrinsèque », essentiellement dû à l'action des UV et à l'agression de facteurs environnementaux comme la pollution et de facteurs dits «intrinsèques » correspondant au vieillissement chronologique, qui démarre dès l'âge de 20 ans.

Le vieillissement affecte d'abord l'épiderme. Le pouvoir de division des kératinocytes dans sa couche basale diminue et le temps de renouvellement de la couche cornée superficielle se rallonge. La maturation de ces cellules est imparfaite et la kératinisation n'aboutit plus à créer une couche cornée régulière et homogène. D'où la diminution de l'épaisseur de l'épiderme, son dessèchement et son aspect rêche.

On note dans le même temps une désorganisation de la partie profonde de la peau. En effet, le derme qui assure à la fois les fonctions fondamentales de cohésion et de nutrition de la peau est la cible principale d'endommagement cutané. En vieillissant, les fibroblastes, à l'origine des substances constitutives du derme, disparaissent ou se transforment en fibrocytes. En conséquence, on assiste à la réduction du taux de la fibronectine qui joue un grand rôle dans l'adhésion et la fonction cellulaire ainsi qu'à la diminution du nombre et de la qualité des fibres élastiques. La diminution de la quantité de collagène (1 % par an) explique la perte d'élasticité du derme et la diminution de l'épaisseur de la peau. La perte de souplesse des liens unissant les cellules dermiques entre elles est à l'origine des différentes plaies et déchirures observables chez les personnes âgées. Ceci explique également l'apparition de poches sous les yeux et de rides cutanées ainsi que l'accentuation des lésions du derme lors de traumatismes minimes.

Le prolongement de la durée de vie associé à un désir de conserver une apparence jeune, ont stimulé la recherche dans la compréhension des changements de structure de la peau lors du vieillissement. En fait, les premiers responsables de notre apparence sont les tissus structurants de la peau (conjonctif, cornéen et fibroplasmique). Parmi leurs constituants, les collagènes, les élastines et les kératines jouent tous un rôle essentiel dans les modifications morphologiques de la peau. C'est en partie sur eux qu'agissent les produits cosmétiques. En effet, il existe une multitude de programmes et traitements "lifting" du visage sur le marché pour réduire les rides et améliorer l'élasticité de la peau. Ceux-ci travaillent la plupart du temps à la surface de la peau ou uniquement sur une protéine-cible mentionnée plus haut et leurs effets sont provisoires et partiellement satisfaisants.

De plus, nombre de composés ne sont pas dénués de toute toxicité, et leur application chronique peut faire apparaître des troubles cutanés tels que l'irritation. Leur procédé d'obtention fait souvent appel, soit à des synthèses chimiques, soit à des extractions à partir des substances naturelles qui sont à la fois longues et coûteuses.

C'est pourquoi, il subsiste toujours le besoin de composés ne présentant pas ces inconvénients (c'est-à-dire non toxiques et faciles à obtenir), et qui ont une action sur plusieurs cibles protéiques simultanément.

De façon surprenante, la Demanderesse a mis en évidence que certains tétrapeptides naturels ou leurs sels permettaient d'agir simultanément à plusieurs niveaux, aussi bien du derme que de l'épiderme. Ces composés présentent de plus l'avantage d'être isolés facilement en ce qui concerne le peptide naturel, ou bien alternativement d'être obtenus par des voies de synthèse peptidiques faciles à mettre en oeuvre et donc peu coûteuses. En outre, de par leur nature peptidique, ces composés présentent une toxicité très faible voire nulle vis-à-vis de l'organisme.

Les peptides ou leurs sels mis en oeuvre dans le cadre de la présente invention, sont dérivés de la structure de base Acétyl-Ser-Asp-Lys-Pro (AcSDKP). Ils sont connus pour leurs propriétés thérapeutiques, en particulier pour la protection de la moelle osseuse pendant les traitements anti-cancéreux par la hématopoïétiques (WO-88/00594 et WO-97/28183). Enfin, plus récemment, la Demanderesse a mis en évidence leurs propriétés angiogéniques (WO-02/24218).

Les documents US-A-5348943, US-5492894 et JP-A-2178207 décrivent l'utilisation de peptides comme des agents actifs sous des compositions cosmétiques antivieillissement.

Aucun des documents de l'art antérieur ne décrit ni ne suggère que ces composés puissent avoir une action anti-vieillissement, ou restructurante de la peau en cosmétique, ni que leur utilisation permette d'avoir un effet positif sur la synthèse des collagènes III et IV, de la fibronectine, des glycosaminoglycanes, et/ou de la kératine.

La présente invention concerne donc l'utilisation cosmétique d'au moins un composé de formule (I) : 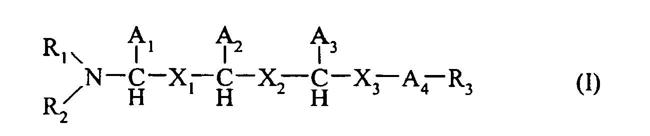 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi l'atome d'hydrogène, les groupements alkyle C₁-C₁₂ linéaire ou ramifié substitué ou non, arylalkyle C₇-C₂₀ substitué ou non, R₄CO- et R₄COO-, R₄ étant un groupement alkyle C₁-C₁₂ linéaire ou ramifié substitué ou non, ou arylalkyle C₇-C₂₀ substitué ou non, parmi les substitutions il faut citer OH, NH₂ ou COOH,
X₁ et X₂ sont des liaisons peptidiques ou pseudopeptidiques,
X₃ est un radical choisi parmi -CO- et -CH₂-, et
R₃ est un groupement choisi parmi -OH, -NH₂, alcoxy C₁-C₁₂ linéaire ou ramifié ou -NH-X₄-CH₂-Z, X₄ étant un groupement hydrocarboné C₁-C₁₂ linéaire ou ramifié, et Z étant un atome d'hydrogène ou un groupement -OH, -CO₂H ou -CONH₂, ainsi que de leurs sels physiologiquement acceptables, dans une composition, en tant qu'agent antivieillissement et restructurant de la peau.

Parmi les groupements alkyles particulièrement adaptés à la mise en oeuvre de la présente invention, on préfèrera les groupements alkyles C₁-C₆ linéaires ou ramifiés. On peut citer plus particulièrement les groupements méthyle, éthyle, propyle, isopropyle, butyle, isobutyle ou tertbutyle.

Par groupement aryle au sens de la présente invention, on entend un groupement carboné aromatique de 6 à 14 atomes de carbone. On peut citer, à titre d'exemple, les groupements phényle, naphtyle ou anthracényle.

Parmi les groupements arylalkyles préférés de l'invention on peut citer les groupements benzyle, et phénéthyle.

Les peptides ou pseudopeptides correspondant à la formule (I) sont dérivés de la structure de base du tétrapeptide Acétyl-Ser-Asp-Lys-Pro (AcSDKP).

Par « radical correspondant à » il faut entendre le radical A de la formule : NH₂-CH(A)-COOH correspondant à l'acide aminé.
Ainsi, A est
-CH₂OH pour Ser,
-CH₂COOH pour Asp,
-CH₂-CH₂-COOH pour Glu,
-(CH₂)₃-NH-C(NH)NH₂ pour Arg,
-(CH₂)₃-NH₂ pour Orn et
-(CH₂)₄-NH₂ pour Lys,
pour l'acide aminé terminal A₄, il s'agit soit de la structure :
=N-CH(A)-CO- ou NH-(CH)A-CO-.

Par « pseudopeptide » on entend désigner des composés similaires aux peptides de référence mais dans lesquels une ou plusieurs liaisons peptidiques -CO-NH- ont été remplacées par une liaison équivalente à la liaison peptidique qui est appelée pseudopeptidique, soit -CH₂-NH-, -CH₂-S-, -CH₂-O-, -CO-CH₂-, -CH₂-CO-, -CH₂-CH₂- représenté par Ψ (CH₂NH) par exemple.

Parmi les radicaux R₁ et R₂, on préférera tout particulièrement l'atome d'hydrogène, ou les radicaux R₄CO- pour lesquels R₄ représente un groupement alkyle C₁-C₃, notamment CH₃CO ainsi que HOOC-CH₂-CH₂-CO-O.
De même, R₃ est de préférence NH₂, OH ou NHCH₃.

Parmi les composés de formule (I) convenant à la mise en oeuvre de l'invention, il faut citer :
CH₃CO-Ser-Asp-Lys-Pro-OH
CH₃CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

Un composé de formule (I) convenant particulièrement bien à la mise en oeuvre de la présente invention est le tétrapeptide naturel CH₃CO-Ser-Asp-Lys-Pro (AcSDKP).

Par le terme « de sel physiologiquement acceptable », on entend au sens de la présente invention tout sel préparé à partir de tout acide non toxique physiologiquement acceptable, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoique, pantothénique, phosphorique, succinique, tartarique et paratoluènesulfonique. Avantageusement, on utilise l'acide chlorhydrique.

Un mode de réalisation de l'invention concerne l'utilisation cosmétique non thérapeutique d'un composé de formule (I) telle que définie précédemment, dans une composition, en tant qu'agent stimulant la production des collagènes III et IV, de la fibronectine, des glycosaminoglycanes, et/ou de la kératine 14 et/ou 19.

Plus particulièrement, la présente invention concerne l'utilisation cosmétique non thérapeutique d'un composé de formule (I) telle que définie précédemment, dans une composition, en tant qu'agent pour lutter contre le vieillissement intrinsèque de la peau.

De façon avantageuse, les composés de formule (I) telle que définie précédemment peuvent être utilisés pour améliorer l'élasticité et/ou la tonicité de la peau, et/ou pour prévenir, diminuer et/ou supprimer l'apparition des rides et ridules sur la peau.

Le tétrapeptide naturel CH₃CO-Ser-Asp-Lys-Pro (AcSDKP) a été isolé de la moelle osseuse de veau foetal (WO-88/00594). Il peut également être obtenu par une synthèse de type peptidique classique. Les peptides ou pseudopeptides de formule (I) apparentés au composé AcSDKP peuvent également être obtenus par une synthèse peptidique ou pseudopeptidique, comme décrit dans le document WO-97/28183.

Les composés de formule (I) sont présents dans les compositions utilisées pour la mise en oeuvre de la présente invention en une quantité comprise entre 0,01% et 10% en poids de préférence entre 0,05 % et 5% en poids, et plus particulièrement entre 0,1% et 2% en poids par rapport au poids total de la composition.

Les compositions cosmétiques utilisées selon l'invention sont destinées à un usage topique, et peuvent se présenter sous toutes les formes galéniques classiquement utilisées pour ce type d'application et notamment sous forme de d'émulsions (huile-dans-eau, eau-dans-huile, émulsion triple huile-dans-eau-dans huile, ou eau-dans-huile-dans-eau), de gels aqueux, de solutions aqueuses, hydroalcooliques, ou huileuses. Elles peuvent être plus ou moins fluides et se présenter sous la forme d'une crème blanche ou colorée, d'une pommade, d'un lait, d'une lotion, d'un sérum, d'une pâte, d'une mousse ou d'un biphase. Elles peuvent aussi se présenter sous forme solide, et par exemple sous forme de stick, ou encore sous forme d'aérosol.

Les compositions utilisées dans le cadre de la présente invention contiennent, outre les composés de formule (I), un ou plusieurs excipients qui peuvent être choisis parmi des composés présentant une bonne compatibilité avec les actifs présents dans la formule. Il s'agit par exemple des polymères hydrosolubles de type polymère naturel, tels les polysaccharides (gomme xanthane, gomme de caroube, peptine...) ou polypeptides, des dérivés cellulosiques type méthylcellulose, hydroxypropylcellulose, hydroxypropyl-méthylcellulose ou encore des polymères synthétiques, polaxamères, carbomères, PVA ou PVP.

Enfin, les compositions de la présente invention peuvent contenir également divers autres excipients de type cosolvant comme l'éthanol, le glycérol, l'alcool benzylique, des humectants (glycérol), des agents facilitant la diffusion (transcurol, urée), ou encore des conservateurs anti-bactériens (p-hydroxybenzoate de méthyle à 0,15%). Elles peuvent également contenir des agents tensioactifs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

Dans un mode de réalisation particulière de l'invention, les composés de formule (I) sont combinés avec au moins un autre principe actif.

La présente invention concerne également un procédé cosmétique non thérapeutique de traitement du vieillissement de la peau comprenant l'application sur la peau d'une composition contenant au moins un composé de formule (1) telle que définie précédemment.

Les exemples qui suivent servent à illustrer l'invention sans toutefois présenter un caractère limitatif.

### Exemple 1 : Etude de l'activité restructurante et anti-âge d'une préparation contenant du tétrapeptide AcSDKP.

Cette étude a été réalisée sur 36 explants de peau humaine obtenue suite à une plastie cutanée mammaire.

### Mode opératoire :

Tous les explants ont été maintenus en survie en milieu de culture pendant 8 jours. AcSDKP à la concentration 10⁻⁵ M ou 10⁻⁸ M dans le sérum physiologique a été appliqué sur les explants par voie topique quotidiennement pendant une semaine. Aux jours 2, 4, 6 et 8 du traitement trois explants de chaque lot (contrôle, +AcSDKP 10⁻⁵ M, +AcSDKP 10⁻⁸ M) ont été prélevés et préparés pour des études histologiques. La description de la morphologie générale ainsi que l'évaluation de l'expression de la kératine basale (CK14) ainsi que de la kératine non différenciée (CK19), du collagène III, du collagène IV, de la fibronectine, et des glycosaminoglycanes n'ayant pas de groupements acides ont été effectués.

### Résultats

### 1. Morphologie générale

Les observations réalisées sur l'ensemble des explants montrent que l'application de AcSDKP aux deux concentrations testées induit, à partir du J6 du traitement, une stimulation de la structure épidermique qui se traduit par une augmentation du nombre d'assises cellulaires vivantes. L'augmentation de l'épaisseur de l'épiderme est plus importante suite à l'application de AcSDKP 10⁻⁵ M (Fig.l).

### 2. Expression de la kératine 14 (CK14) et de la kératine 19 (CK19)

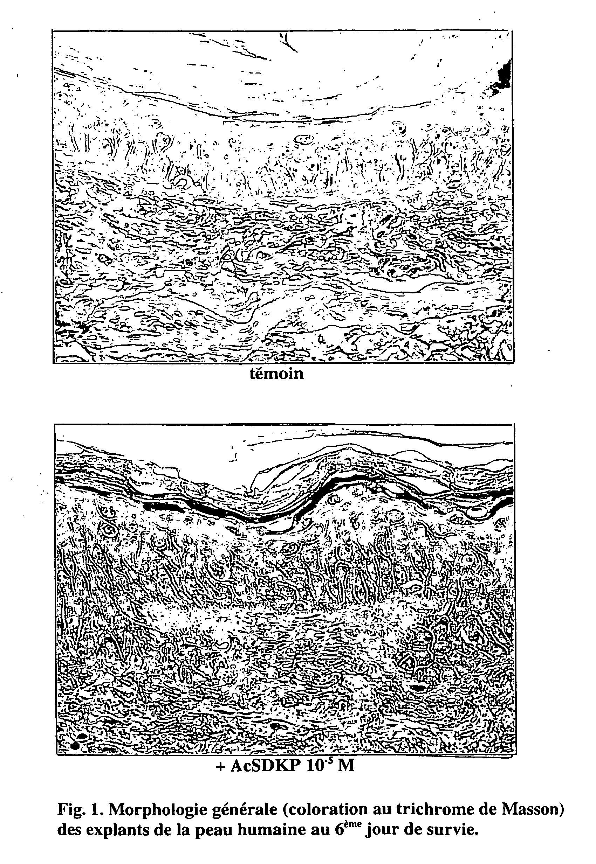

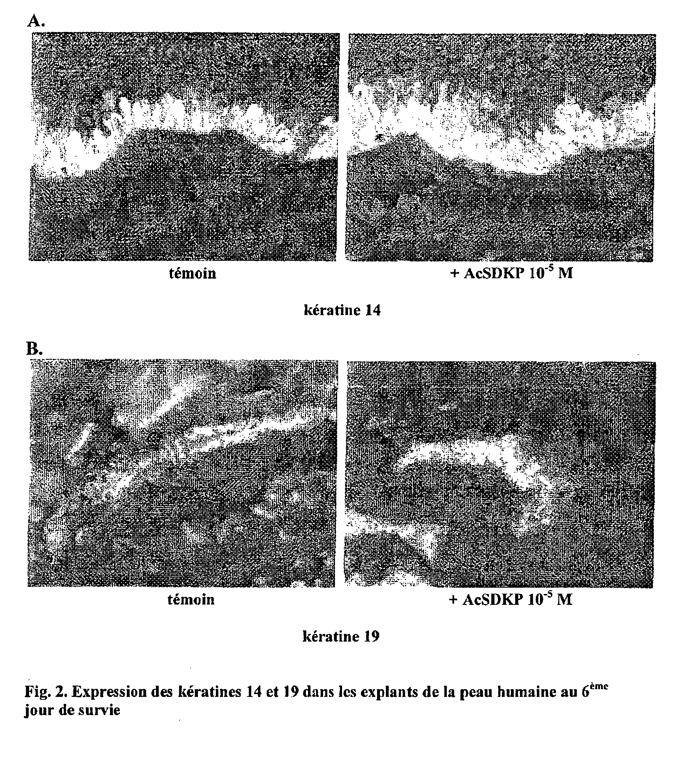

L'immunomarquage de la CK14 a révélé une augmentation progressive du taux de cette protéine en position basale et nettement supra-basale à partir du J4 du traitement. L'activité restructurante visualisée est plus importante avec AcSDKP 10⁻⁵ M (Fig. 2A). L'immunomarquage de la CK19 a permis de visualiser sa surexpression sur les annexes dermiques de la peau à partir de J6 du traitement avec AcSDKP aux deux concentrations testées ; l'effet de AsSDKP utilisé à la concentration de 10⁻⁵ M est plus prononcé (Fig. 2B).
AcSDKP stimule donc la production de la CK14 et de la CK19 dans la peau traitée.

### 3. Expression du collagène dermique (type III) et du collagène de membrane basale (type IV)

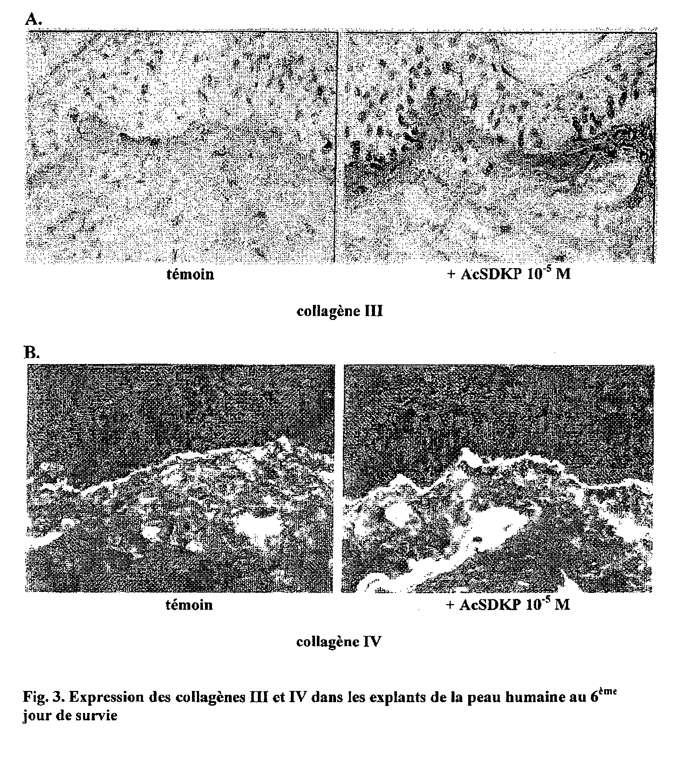

Après 6 jours de traitement avec AcSDKP à 10⁻⁵ M on observe une très nette augmentation du collagène III dans le derme papillaire le long de la jonction dermo-épidermique (Fig. 3A). Avec AcSDKP à 10⁻⁸ M cette surexpression est plus modérée le long de la jonction dermo-épidermique, mais le collagène III apparaît plus présent dans le reste du derme papillaire et dans le derme réticulaire. A J8, une très nette surexpression du collagène III est observée dans le derme papillaire et réticulaire des explants soumis au traitement avec AcSDKP 10⁻⁵ M. Avec AcSDKP à 10⁻⁸ M, le taux du collagène III augmente fortement le long de la jonction dermo-épidermique formant une bande de collagène plus dense.

L'augmentation du taux du collagène de membrane basale de type IV le long de la jonction dermo-épidermique à J6 et à J8, dans la peau traitée avec AcSDKP aux deux concentrations testées, est plus modérée que celle du collagène de type III et plus importante pour AcSDKP 10⁻⁵ M (Fig. 3B).

L'immunomarquage des collagènes III et IV a révélé une activité stimulante de AcSDKP sur l'expression de ces composés dans la peau traitée.

### 4. Expression de la fibronectine

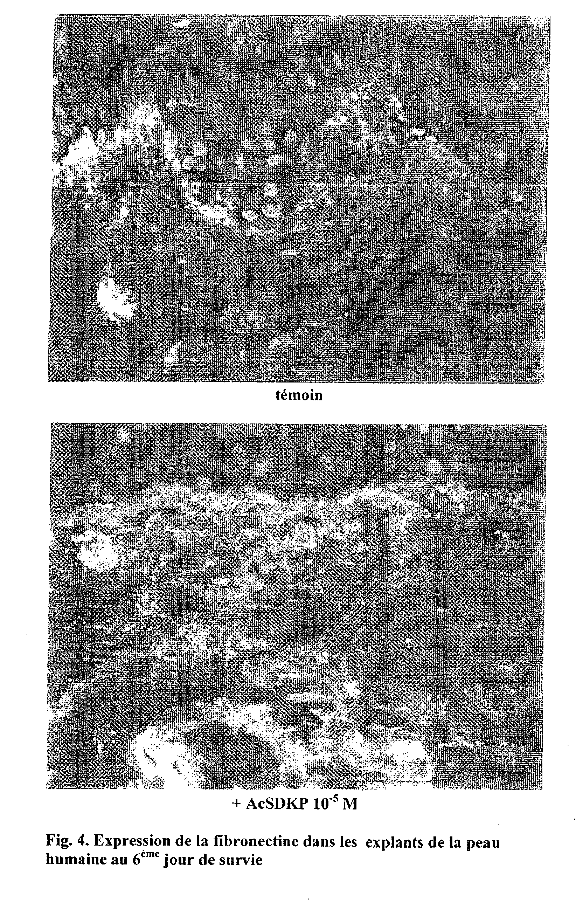

Sous le disque d'application de AcSDKP, le réseau de fibronectine est plus dense avec des fibres plus épaisses. Cette activité est plus importante pour AcSDKP testé à la concentration 10⁻⁵ M (Fig. 4).

L'immunomarquage de la fibronectine montre que AcSDKP induit une forte surexpression de cette protéine le long de la jonction dermo-épidermique ainsi que dans le derme papillaire et réticulaire de la peau traitée.

### 5. Expression des glycosaminoglycanes (GAGs)

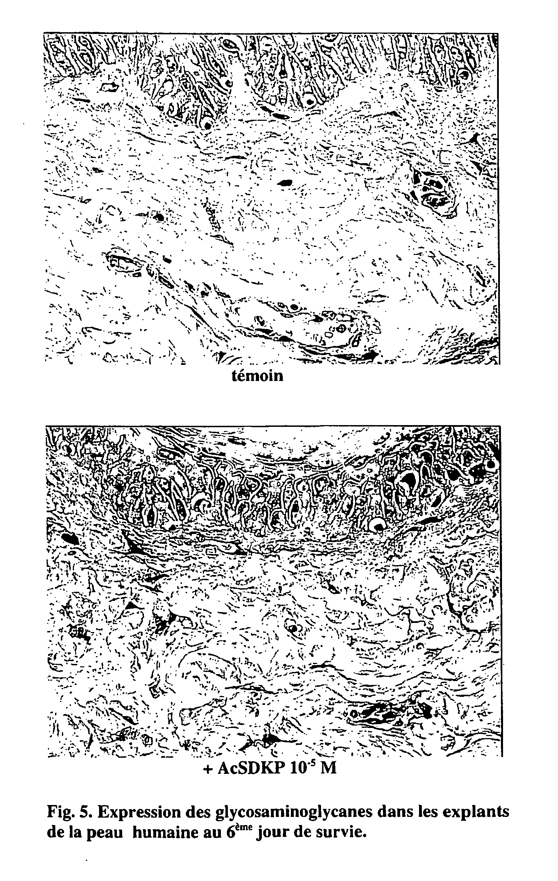

Une coloration des coupes histologiques des explants de la peau au bleu alcian P.A.S. spécifique des GAGs sans groupements acides a permis de mettre en évidence une augmentation significative de ces composants de la peau suite au traitement des explants avec AcSDKP aux deux concentrations testées. Cet effet, bien visible à J6 et à J8, est caractérisé par une augmentation du nombre de fibroblastes nettement P.A.S. positifs dans le derme papillaire, surtout dans une bande située le long de la jonction dermo-épidermique et par une augmentation des GAGs dans ces fibroblastes. AcSDKP appliqué à la concentration 10⁻⁵ M manifeste une activité plus importante que lorsque le tétrapeptide est utilisé à une concentration de 10⁻⁸ M (Fig. 5).

### Conclusions

L'ensemble des observations ci-dessus traduit la restructuration de l'épiderme et du derme sous l'action du tétrapeptide AcSDKP. Ce type de modifications représente les critères d'une peau plus jeune.

Les formulations suivantes sont préparées de façon classique par l'homme du métier.

### Exemple 2 : Emulsion H/E

| | | |
|---|---|---|
| Polyéthylène glycol oxyéthyléné par 50 moles d'oxyde d'éthylène | | 3% |
| Monodiglycérylstéarate | | 3% |
| Huile de vaseline | | 3% |
| Alcool cétylique | | 5% |
| AcSDKP | | 2% |
| Eau | qsp | 100% |

### Exemple 3 :

| | | |
|---|---|---|
| Octylpalmitate | | 10% |
| Glycérylisostéarate | | 4% |
| Huile de vaseline | | 10% |
| Sorbitol | | 2% |
| Vitamine E | | 1% |
| AcSDKP | | 0,5% |
| Glycérol | | 3% |
| Eau | qsp | 100% |

## Revendications

1. Utilisation cosmétique non thérapeutique d'au moins un composé de formule (I) : 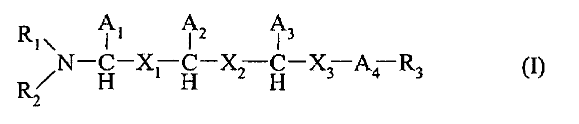 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi l'atome d'hydrogène, les groupements alkyle C₁-C₁₂ linéaire ou ramifié substitué ou non, arylalkyle C₇-C₂₀ substitué ou non, R₄CO- et R₄COO-, R₄ étant un groupement alkyle C₁-C₁₂ linéaire ou ramifié substitué ou non, ou arylalkyle C₇-C₂₀ substitué ou non, parmi les substitutions il faut citer OH, NH₂ ou COOH,
X₁ et X₂ sont des liaisons peptidiques ou pseudopeptidiques,
X₃ est un radical choisi parmi -CO- et -CH₂-, et
R₃ est un groupement choisi parmi -OH, -NH₂, alcoxy C₁-C₁₂ linéaire ou ramifié ou -NH-X₄-CH₂-Z, X₄ étant un groupement hydrocarboné C₁-C₁₂ linéaire ou ramifié, et Z étant un atome d'hydrogène ou un groupement -OH, - CO₂H ou -CONH₂,
ainsi que de leurs sels physiologiquement acceptables, dans une composition, en tant qu'agent antivieillissement de la peau.

2. Utilisation cosmétique non thérapeutique d'au moins un composé de formule (I) : 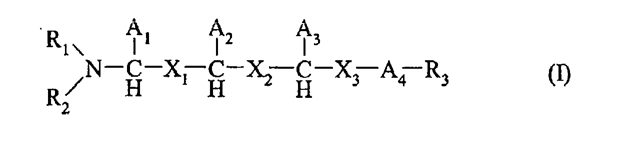 dans laquelle,
A₁ est le radical correspondant à D- ou L- Ser,
A₂ est le radical correspondant à D- ou L- Asp ou Glu,
A₃ est le radical correspondant à D- ou L- Lys, Arg ou Orn,
A₄ est le radical correspondant à D- ou L- Pro,
R₁ et R₂ sont choisis indépendamment parmi l'atome d'hydrogène, les groupements alkyle C₁-C₁₂ linéaire ou ramifié substitué ou non, arylalkyle C₇-C₂₀ substitué ou non, R₄CO- et R₄COO-, R₄ étant un groupement alkyle C₁-C₁₂ linéaire ou ramifié substitué ou non, ou arylalkyle C₇-C₂₀ substitué ou non, parmi les substitutions il faut citer OH, NH₂ ou COOH,
X₁ et X₂ sont des liaisons peptidiques ou pseudopeptidiques,
X₃ est un radical choisi parmi -CO- et -CH₂-, et
R₃ est un groupement choisi parmi -OH, -NH₂, alcoxy C₁-C₁₂ linéaire ou ramifié ou -NH-X₄-CH₂-Z, X₄ étant un groupement hydrocarboné C₁-C₁₂ linéaire ou ramifié, et Z étant un atome d'hydrogène ou un groupement -OH, - CO₂H ou -CONH₂,
ainsi que de leurs sels physiologiquement acceptables, dans une composition, en tant qu'agent restructurant de la peau.

3. Utilisation cosmétique d'un composé de formule (I) telle que définie dans la revendication 1, dans une composition, en tant qu'agent stimulant la production des collagènes III et IV, de la fibronectine, des glycosaminoglycanes, et/ou de la kératine 14 et/ou 19.

4. Utilisation cosmétique d'un composé de formule (I) telle que définie dans la revendication 1, dans une composition, en tant qu'agent pour lutter contre le vieillissement intrinsèque de la peau.

5. Utilisation selon l'une des revendications 1 à 4, pour améliorer l'élasticité et/ou la tonicité de la peau.

6. Utilisation selon l'une des revendications 1 à 4, pour prévenir, diminuer et/ou supprimer l'apparition des rides et ridules sur la peau.

7. Utilisation selon l'une des revendications 1 à 6, **caractérisée en ce que** le dérivé de formule (I) comporte au moins une liaison pseudopeptidique.

8. Utilisation selon l'une des revendications 1 à 7, **caractérisée en ce que** le composé de formule (I) est choisi parmi :
CH₃CO-Ser-Asp-Lys-Pro-OH
CH3CO-Ser-Ψ-(CH₂NH)-Asp-Lys-Pro-OH
CH3CO-Ser-Asp-Ψ-(CH₂NH)-Lys-Pro-OH
CH3CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH2NH)-Asp-Lys-Pro-OH
HOOCCH₂CH₂CO-Ser-Asp-Ψ-(CH2NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

9. Utilisation selon l'une des revendications 1 à 8, **caractérisée en ce que** le composé de formule (I) est représenté par la formule : CH₃CO-Ser-Asp-Lys-Pro-OH.

10. Utilisation selon l'une des revendications 1 à 9, **caractérisée en ce que** le composé de formule (I) est présent dans la composition en une quantité comprise entre 0,01 % et 10 % en poids par rapport au poids total de la composition.

11. Procédé cosmétique non thérapeutique de traitement du vieillissement de la peau comprenant l'application sur la peau d'une composition contenant au moins un composé de formule (I) telle que définie dans la revendication 1.

## Claims

1. Cosmetic, non-therapeutic use of at least one compound of formula (I): 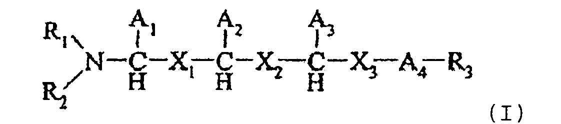 wherein
A₁ is the radical corresponding to D- or L-Ser,
A₂ is the radical corresponding to D- or L-Asp or Glu,
A₃ is the radical corresponding to D- or L-Lys, Arg or Orn,
A₄ is the radical corresponding to D- or L-pro,
R₁ and R₂ are independently chosen from among the hydrogen atom, a substituted or nonsubstituted C₁-C₁₂ linear or branched alkyl group, a substituted or nonsubstituted C₇-C₂₀ linear or branched arylalkyl group, R₄CO- and R₄COO- wherein R₄ is a substituted or nonsubstituted C₁-C₁₂ linear or branched alkyl group, or a substituted or nonsubstituted C₇-C₂₀ arylalkyl group. Substitutions include OH, NH₂ or COOH,
X₁ and X₂ are peptide and pseudopeptide bonds,
X₃ is a radical chosen among -CO- and -CH2-, and
R₃ is a group chosen from among -OH, -NH₂, C₁-C₁₂ linear or branched alcoxy or -NH-X₄-CH₂-Z wherein X₄ is a C₁-C₁₂ linear or branched hydrocarbon group and Z is a hydrogen atom or -OH, -CO₂H or -CONH₂ group,
as well as their physiologically acceptable salts, in a composition as a skin anti-aging agent.

2. Cosmetic, non-therapeutic use of at least one compound of formula (I): 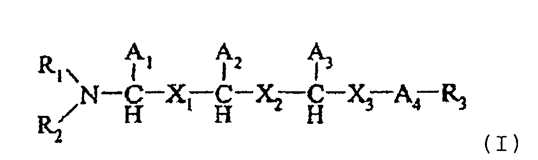 wherein
A₁ is the radical corresponding to D- or L-Ser,
A₂ is the radical corresponding to D- or L-Asp or Glu,
A₃ is the radical corresponding to D- or L-Lys, Arg or Orn,
A₄ is the radical corresponding to D- or L-pro,
R₁ and R₂ are independently chosen from among the hydrogen atom, a substituted or nonsubstituted C₁-C₁₂ linear or branched alkyl group, a substituted or nonsubstituted C₇-C₂₀ linear or branched arylalkyl group, R₄CO- and R₄COO- wherein R₄ is a substituted or nonsubstituted C₁-C₁₂ linear or branched alkyl group, or a substituted or nonsubstituted C₇-C₂₀ arylalkyl group. Substitutions include OH, NH₂ or COOH,
X₁ and X₂ are peptide and pseudopeptide bonds,
X₃ is a radical chosen among -CO- and -CH2-, and
R₃ is a group chosen from among -OH, -NH₂, C₁-C₁₂ linear or branched alcoxy or -NH-X₄-CH₂-Z wherein X₄ is a C₁-C₁₂ linear or branched hydrocarbon group and Z is a hydrogen atom or -OH, -CO₂H or -CONH₂ group,
as well as their physiologically acceptable salts, in a composition as a skin restructuring agent.

3. Cosmetic use of a compound of formula (I) as defined in claim 1 in a composition as an agent to stimulate the production of collagen III and IV, fibronectin, glycosaminoglycans and/or keratin 14 and/or 19.

4. Cosmetic use of a compound of formula (I) as defined in claim 1 in a composition as an agent to prevent intrinsic skin aging.

5. Use as claimed in claims 1 to 4, to improve skin elasticity and/or tonicity.

6. Use as claimed in claims 1 to 4, to prevent, reduce, and/or suppress the appearance of wrinkles and crow's-feet on the skin.

7. Use as claimed in claims 1 to 6, wherein the derivative of formula (I) includes at least one pseudopeptide bond.

8. Use as claimed in claims 1 to 7, wherein the compound of formula (I) is chosen from among:
CH₃CO-Ser-Asp-Lys-Pro-OH
CH3CO-Ser-Ψ-(CH₂NH)-Asp-Lys-Pro-OH
CH₃CO-Ser-Asp-Ψ-(CH₂NH)-Lys-Pro-OH
CH₃CO-Ser-Asp-Lys-Ψ-(CH₂N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH₃CO-Ser-Asp-Lys-Ψ-(CH₂N)-Pro-NH₂
H-Ser-Ψ-(CH₂NH)-Asp-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂ CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

9. Use as claimed in claims 1 to 8, wherein the compound of formula (I) is represented by the formula: CH₃CO-Ser-Asp-Lys-Pro-OH.

10. Use as claimed in claims 1 to 9, wherein the compound of formula (I) is present in the composition in an amount ranging from 0.01% to 10% by weight with respect to the total weight of the composition.

11. Cosmetic, non-therapeutic process for the treatment of skin aging comprising the application to the skin of a composition containing at least one derivative of formula (I) as defined in claim 1.

## Patentansprüche

1. Nicht-therapeutische kosmetische Verwendung
mindestens einer Verbindung der Formel (I) 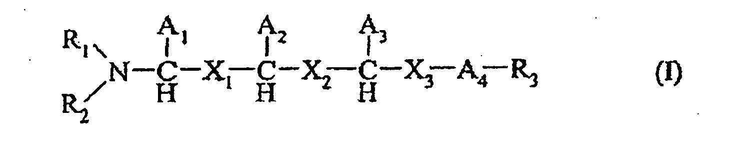 in der
A₁ das Radikal D- oder L-Ser entsprechende Radikal ist,
A₂ das D- oder L-Asp oder Glu entsprechende Radikal ist,
A₃ das D- oder L-Lys, Arg oder Orn entsprechende Radikal ist,
A₄ das D- oder L-Pro entsprechende Radikal ist,
R₁ und R₂ unabhängig aus dem Wasserstoffatom, den linearen oder verzweigten substituierten oder nicht substituierten C₁-C₁₂-Alkylgruppen, den substituierten oder nicht substituierten C₇-C₂₀-Arylalkylgruppen, R₄CO- und R₄COO- ausgewählt sind, wobei R₄ eine lineare oder verzweigte substituierte oder nicht substituierte C₁-C₁₂-Alkylgruppe ist oder eine substituierte oder nicht substituierte C₇-C₂₀-Arylalkylgruppe, wobei unter den Substitutionen OH, NH₂ oder COOH zu nennen wären,
X₁ und X₂ Peptid- oder Pseudopeptidverbindungen sind,
X₃ ein aus -CO- und -CH₂- ausgewähltes Radikal ist, und
R₃ eine Gruppe ist, die aus -OH, -NH₂, linearem oder verzweigtem C₁-C₁₂-Alcoxy oder -NH-X₄-CH₂-Z ausgewählt ist, wobei X₄ eine hydrocarbonierte lineare oder verzeigte C₁-C₁₂-Gruppe ist, und Z ein Wasserstoffatom oder eine -OH, -CO₂H- oder-CONH₂-Gruppe ist,
sowie aus ihren physiologisch verträglichen Salzen in einer Zusammensetzung als Wirkstoff gegen die Hautalterung.

2. Nicht-therapeutische kosmetische Verwendung mindestens einer Verbindung der Formel (I): 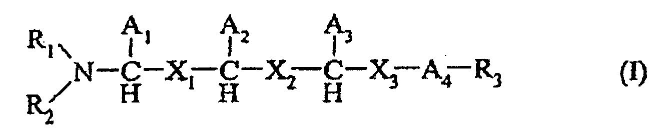 in der
A₁ das Radikal D- oder L-Ser entsprechende Radikal ist,
A₂ das D- oder L-Asp oder Glu entsprechende Radikal ist,
A₃ das D- oder L-Lys, Arg oder Orn entsprechende Radikal ist,
A₄ das D- oder L-Pro entsprechende Radikal ist,
R₁ und R₂ unabhängig aus dem Wasserstoffatom, den linearen oder verzweigten substituierten oder nicht substituierten C₁-C₁₂-Alkylgruppen, den substituierten oder nicht substituierten C₇-C₂₀-Arylalkylgruppen, R₄CO- und R₄COO- ausgewählt sind, wobei R₄ eine lineare oder verzweigte substituierte oder nicht substituierte C₁-C₁₂-Alkylgruppe ist oder eine substituierte oder nicht substituierte C₇-C₂₀-Arylalkylgruppe, wobei unter den Substitutionen OH, NH₂ oder COOH zu nennen wären,
X₁ und X₂ Peptid- oder Pseudopeptidverbindungen sind,
X₃ ein aus -CO- und -CH₂- ausgewähltes Radikal ist, und
R₃ eine Gruppe ist, die aus -OH, -NH₂, linearem oder verzweigtem C₁-C₁₂-Alcoxy oder -NH-X₄-CH₂-Z ausgewählt ist, wobei X₄ eine hydrocarbonierte lineare oder verzeigte C₁-C₁₂-Gruppe ist, und Z ein Wasserstoffatom oder eine -OH,-CO₂H- oder-CONH₂-Gruppe ist,
sowie aus ihren physiologisch verträglichen Salzen in einer Zusammensetzung als Wirkstoff zur Restrukturierung der Haut.

3. Kosmetische Verwendung einer Verbindung der Formel (I), so wie in Anspruch 1 definiert, in einer Verbindung als Wirkstoff, der die Produktion der Kollagene III und IV, von Fibronectin, der Glycosaminoglycane und/oder des Keratins 14 und/oder 19 stimuliert.

4. Kosmetische Verwendung einer Verbindung der Formel (I), so wie in Anspruch 1 definiert, in einer Verbindung als Wirkstoff zur Bekämpfung der inhärenten Hautalterung.

5. Verwendung nach einem der Ansprüche 1 bis 4 zur Verbesserung der Elastizität und/oder der Spannkraft der Haut.

6. Verwendung nach einem der Ansprüche 1 bis 4 zur Vorbeugung, Verringerung und/oder Unterdrückung des Auftretens von Falten und Fältchen auf der Haut.

7. Verwendung nach einem der Ansprüche 1 bis 6, **dadurch gekennzeichnet, dass** das Derivat der Formel (I) mindestens eine Pseudopeptidverbindung aufweist.

8. Verwendung nach einem der Ansprüche 1 bis 7, **dadurch gekennzeichnet, dass** die Verbindung der Formel (I) ausgewählt ist aus:
CH₃CO-Ser-Asp-Lys-Pro-OH
CH3CO-Ser-Ψ-(CH2NH)-Asp-Lys-Pro-OH
CH₃CO-Ser-Asp-Ψ-(CH₂NH)-Lys-Pro-OH
CH3CO-Ser-Asp-Lys-Ψ-(CH2N)-Pro-OH
CH₃CO-Ser-Ψ-(CH₂NH)-Asp-Lys-Pro-NH₂
CH₃CO-Ser-Asp-Ψ-(CH₂NH)-Lys-Pro-NH₂
CH3CO-Ser-Asp-Lys-Ψ-(CH₂N)-PrO-NH₂
H-Ser-Ψ-(CH₂NH)-AsP-Lys-Pro-OH
H-Ser-Asp-Ψ-(CH₂NH)-Lys-Pro-OH
H-Ser-Asp-Lys-Ψ-(CH₂N)-Pro-OH
HOOCCH₂CH₂CO-Ser-Ψ-(CH₂NH)-Asp-Lys-Pro-OH
HOOCCH₂CH₂CO-Ser-Asp-Ψ-(CH₂NH)-Lys-Pro-OH
HOOCCH₂CH₂CO-Ser-Asp-Lys-Ψ-(CH₂N)-Pro-OH
H-Ser-Ψ-(CH₂NH)-Asp-Lys-Pro-NH₂
H-Ser-Asp-Ψ-(CH₂NH)-Lys-Pro-NH₂
H-Ser-Asp-Lys-Ψ-(CH₂N)-Pro-NH₂
HOOCCH₂CH₂CO-Ser-Ψ-(CH₂NH)-Asp-Lys-Pro-NH₂
HOOCCH₂CH₂CO-Ser-Asp-Ψ-(CH₂NH)-Lys-Pro-NH₂
HOOCCH₂CH₂CO-Ser-Asp-Lys-Ψ-(CH₂N)-Pro-NH₂
CH₃CO-Ser-Asp-Lys-Pro-NH₂
H-Ser-Asp-Lys-Pro-NH₂
CH₃CO-Ser-Asp-Lys-Pro-NHCH₃
H-Ser-Asp-Lys-Pro-NHCH₃
HOOCCH₂CH₂CO-Ser-Asp-Lys-Pro-NHCH₃
HOOCCH₂CH₂CO-Ser-Asp-Lys-Pro-NH₂.

9. Verwendung nach einem der Ansprüche 1 bis 8, **dadurch gekennzeichnet, dass** die Verbindung der Formel (I) durch die Formel CH₃CO-Ser-Asp-Lys-Pro-OH dargestellt ist.

10. Verwendung nach einem der Ansprüche 1 bis 9, **dadurch gekennzeichnet, dass** die Verbindung der Formel (I) in der Zusammensetzung in einer Menge zwischen 0,01 Gew.-% und 10 Gew.-% im Verhältnis zum Gesamtgewicht der Zusammensetzung vorhanden ist.

11. Nicht-therapeutisches kosmetisches Verfahren zur Behandlung der Hautalterung, das das Auftragen auf die Haut einer Zusammensetzung umfasst, die mindestens eine Verbindung der Formel (I) enthält, so wie in Anspruch 1 definiert.
